(19) Europäisches Patentamt
European Patent Office
Office européen des brevets

(11) **EP 2 858 613 B1**

(12) **EUROPÄISCHE PATENTSCHRIFT**

(45) Veröffentlichungstag und Bekanntmachung des Hinweises auf die Patenterteilung:
**14.03.2018 Patentblatt 2018/11**

(51) Int Cl.:
***A61F 13/02*** *(2006.01)*

(21) Anmeldenummer: **13727518.6**

(22) Anmeldetag: **28.05.2013**

(86) Internationale Anmeldenummer:
**PCT/EP2013/001571**

(87) Internationale Veröffentlichungsnummer:
**WO 2013/182279 (12.12.2013 Gazette 2013/50)**

(54) **MEDIZINPRODUKT ZUR VERSORGUNG EINES INDIVIDUUMS**

MEDICINAL PRODUCT FOR THE CARE OF AN INDIVIDUAL

DISPOSITIF MÉDICAL SERVANT À SOIGNER UNE PERSONNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.06.2012 DE 102012011422**

(43) Veröffentlichungstag der Anmeldung:
**15.04.2015 Patentblatt 2015/16**

(73) Patentinhaber: **Gottlieb Binder Gmbh & Co. Kg**
**71084 Holzgerlingen (DE)**

(72) Erfinder: **TUMA, Jan**
**71083 Herrenberg (DE)**

(74) Vertreter: **Bartels und Partner, Patentanwälte**
**Lange Strasse 51**
**70174 Stuttgart (DE)**

(56) Entgegenhaltungen:
**WO-A1-2005/087033      US-A1- 2005 148 984**
**US-A1- 2009 216 170**

## Beschreibung

Die Erfindung betrifft ein Medizinprodukt zur Versorgung eines mindestens eine, insbesondere großflächige, Verletzung, wie eine Brandwunde oder eine Hautabschürfung, aufweisenden Individuums, mit den Merkmalen im Oberbegriff von Anspruch 1.

Ein derartiges Medizinprodukt ist beispielsweise aus EP 1 190 723 A2 bekannt. Das bekannte Medizinprodukt ist ein Wundverband umfassend eine Trägerfolie, auf welche vollflächig eine Klebeschicht aufgetragen ist, wobei die Klebeschicht aus Polyacrylat oder Kautschuk besteht und in Randbereichen der Trägerfolie eine Klebe- bzw. Funktionsfläche zur Anhaftung am Patienten ausbildet. Der Wundverband weist einen auf eine Wunde des Patienten aufzulegenden Wundversorgungsbereich auf Basis von Hydrokolloiden als weitere Funktionsfläche auf. Im Wundversorgungsbereich wird aufgrund der Hydrokolloide ein feuchtes Wundheilungsmilieu generiert, welches die Wunde nicht austrocknen lässt und ein optimales Milieu zur schnellen Wundheilung erzeugt.

Um ein Austrocknen des feuchten Wundheilungsmilieus zu verhindern und die zu behandelnde Wunde bzw. Verletzung frei von äußeren Kontaminationen zu halten, ist eine gute, einen Feuchtigkeitsdurchtritt unterbindende Anhaftung des Medizinprodukts am Individuum, insbesondere an einem Patienten, erforderlich. Demgegenüber steht das Erfordernis, das Medizinprodukt beispielsweise beim Verbandswechsel wieder vom Patienten abzunehmen, wobei der Patient weder verletzt noch allzu großen Schmerzen ausgesetzt werden soll. Insbesondere bei Patienten mit Brandwunden sind die die Brandwunde umgebenden Wundrandbereiche sehr empfindlich, so dass es bei der Abnahme eines dort anhaftenden Medizinprodukts neben starken Schmerzen für den Patienten zur Ablösung bereits nachgebildeter Hautschichten kommen kann.

Die US 2005/148984 A1 beschreibt ein Medizinprodukt zur Versorgung eines mindestens eine, insbesondere großflächige, Verletzung, wie eine Brandwunde oder eine Hautabschürfung, aufweisenden Individuums, umfassend mindestens eine Funktionsfläche zur zumindest teilweisen Abdeckung der jeweiligen Verletzung und/oder zur Festlegung des Medizinprodukts am Individuum, wobei mindestens eine Funktionsfläche von ihr vorstehende Stielteile aufweist, deren freien stirnseitigen Enden derart ein Anhaftteil ausbilden, dass dieses zumindest teilweise am Individuum und/ oder an einer weiteren Funktionsfläche überwiegend mittels Van-der-Waals-Kräften anhaftbar ist, wobei das Medizinprodukt mindestens eine Funktionsfläche zur Versorgung und/oder zur Anlage an der jeweiligen Verletzung des Individuums, wie an einem Wundbereich, aufweist.

Die Erfindung stellt sich die Aufgabe, ein Medizinprodukt dahingehend zu verbessern, dass eine gute Festlegung, insbesondere Anhaftung, des Medizinprodukts am Individuum über die mindestens eine Funktionsfläche sichergestellt ist und zugleich eine verletzungsfreie, für den Patienten möglichst schmerzlose Wiederabnahme des Medizinprodukts vom Patienten ermöglicht ist.

Diese Aufgabe wird gelöst durch ein Medizinprodukt mit den Merkmalen des Patentanspruchs 1 in seiner Gesamtheit.

Ein erfindungsgemäßes Medizinprodukt zeichnet sich dadurch aus, dass die Funktionsfläche zur Versorgung von einer fluiddurchlässigen, vorzugsweise perforierten, Funktionslage überdeckt ist und dass die Funktionslage an ihrer der jeweiligen Verletzung zugewandten Seite eine Funktionsfläche mit Stielteilen zur Ausbildung eines an der jeweiligen Verletzung anhaftenden Anhaftteils aufweist. In Abhängigkeit der zu versorgenden Verletzung weist die Funktionsfläche Stoffe zur Erzeugung eines feuchten Wundheilungsmilieus, zur Aufnahme von Wundsekret, zur Förderung von Heilungsprozessen im Bereich der Verletzung, zur Polsterung, zur Erwärmung oder Kühlung der Verletzung, etc. auf. Es ist ferner vorgesehen, dass mindestens eine Funktionsfläche von ihr vorstehende Stielteile aufweist, deren freien stirnseitigen Enden derart ein Anhaftteil ausbilden, dass dieses zumindest teilweise am Individuum und/oder an einer weiteren Funktionsfläche überwiegend mittels Van-der-Waals-Kräften anhaftbar ist.

Van-der-Waals-Kräfte sind nach Van der Waals benannte zwischenmolekulare Kräfte, die als schwache Bindungskräfte zwischen inerten Atomen und gesättigten Molekülen auftreten. Während bei der Wechselwirkung zwischen Atomen lediglich die sog. Dispersionskräfte zum Tragen kommen, sind bei Molekülen die Wechselwirkungen induzierter bzw. eventuell vorhandener permanenter Dipolmomente (Orientierungseffekt) als zusätzliche Anziehungskräfte wirksam. Es sei darauf hingewiesen, dass zwar von manchen Autoren Van-der-Waals-Kräfte als Synonym zu zwischenmolekularen Kräften angesprochen werden, dass aber mehrheitlich unter Van-der-Waals-Kräften solche sehr weitreichenden Anziehungskräfte zwischen neutralen Molekülen verstanden werden, deren Energie mit der 6. Potenz des Molekülabstands abnimmt. Die Kräfte sind beispielsweise wirksam in Wirt-Gast-Beziehungen, in Molekülgitter-Kristallen, Einschlussverbindungen, Molekülverbindungen und bei Phänomenen der Kolloidchemie, der Grenzflächen- und Oberflächenchemie, etc. zu beobachten (vgl. RÖMPPS CHEMIE LEXIKON, 8. Aufl., Franckh'sche Verlagshandlung Stuttgart).

Die Anhaftung des stirnseitigen Endes eines Stielteils mittels Van-der-Waals-Kräfte findet eine Entsprechung in der Natur, beispielsweise bei den Füßen eines Geckos, der in der Lage ist, aufgrund der Fußgestaltung unterhalb einer Decke oder auch entlang von senkrecht verlaufenden Glasflächen sich zu bewegen. Die Stiele, die in millionenfacher Anordnung bei einem Gecko-Fuß vorhanden sind, werden dabei in der Fachsprache mit "Seta" bezeichnet und die sich am freien Stielende anschließenden Einzelfasern oder Einzelfilamente als "Spatula". Dadurch, dass bei dem erfindungsgemäßen Medizinprodukt gegenüber der jeweiligen Funktionsfläche vorstehende Stielteile ausgebildet sind, deren Anhaftung überwiegend mittels der genannten Van-der-Waals-Kräfte realisiert wird, werden die sehr guten Anhaftwerte eines Gecko-Fußes als biomechanisches Modell erreicht.

Die Anhaftung aufgrund von Van-der-Waals-Kräften zwischen der jeweiligen Funktionsfläche des Medizinprodukts und beispielsweise einer entsprechenden Anlagefläche am Körper, typischerweise an der Haut, des Individuums, wie eines Patienten, ist derart stark ausgebildet, dass ein Feuchtigkeitsdurchtritt durch die jeweilige anhaftende Funktionsfläche weitestgehend verhindert wird und zugleich die Funktionsfläche mit geringem Kraftaufwand wieder vom Patienten gelöst und das Medizinprodukt als Ganzes abgenommen werden kann. Die am einzelnen Stielteil ausgebildeten Van-der-Waals-Kräfte sind so gering, dass beim Ablösen der Funktionsfläche von der Anlagefläche am Körper des Patienten keinerlei unerwünschte Verletzungen, wie die Abnahme von neu gebildeten Hautschichten, entstehen und die Wiederabnahme des Medizinprodukts mit vergleichsweise geringen Schmerzen, optimalerweise schmerzlos, für einen Patienten durchgeführt werden kann.

Alternativ zur unmittelbaren Anhaftung am Individuum kann das erfindungsgemäße Medizinprodukt derart am Individuum fixiert werden, dass die jeweilige Funktionsfläche an einer weiteren Funktionsfläche anhaftet, wodurch das am entsprechenden Körperteil des Individuums anliegende Medizinprodukt in der gewählten Lage fixiert ist. An der weiteren Funktionsfläche können auch Stielteile zur Ausbildung eines weiteren überwiegend mittels Van-der-Waals-Kräften anhaftbaren Anhaftteils ausgebildet sein. Es ist jedoch auch denkbar, an der weiteren Funktionsfläche eine Art Vlies aus mindestens einem Schlaufenmaterial, vorzugsweise jedoch hermaphroditische Haftelemente oder ein anderes geeignetes Befestigungsmaterial zum Zusammenwirken mit den Stielteilen der einen Funktionsfläche auszubilden. Ferner besteht die Möglichkeit der Anhaftung an glatten Flächen des Kunststoffabdeckbandes selbst.

In einer bevorzugten Ausführungsform des erfindungsgemäßen Medizinprodukts weist dieses einen Träger auf, welcher mindestens eine Funktionsfläche aufweist und als vorzugsweise bandartiges Flächengebilde ausgebildet ist. Ein Träger mit der mindestens einen die Stielteile aufweisenden Funktionsfläche lässt sich in großindustriellem Maßstab sehr kostengünstig herstellen und entsprechend des geometrisch gewünschten Medizinprodukts zuschneiden. Ein bandartiges Medizinprodukt lässt sich in einfacher Weise durch Umwickeln des entsprechenden Körperteils, beispielsweise eines Arms oder eines Fingers, am Individuum, wie am Patienten, fixieren. Durch mindestens zwei Stielteile aufweisende Funktionsflächen, die an den Enden des bandartigen Medizinprodukts zweckmäßigerweise auf der in Gebrauch dem Patienten zugewandten Seite und auf der im Gebrauch dem Patienten abgewandten Seite vorgesehen sind, lässt sich in einfacher Weise eine wieder lösbare Verbindung der beiden Funktionsflächen und auf diese Weise eine wieder lösbare Festlegung bzw. Fixierung des Medizinprodukts am Patienten sicherstellen. Der Träger kann an seiner im Gebrauch dem Patienten zugewandten Seite vollflächig mit einer Stielteile aufweisenden Funktionsfläche versehen sein, es ist jedoch auch denkbar, nur Teilbereiche des Trägers mit einer derartigen Funktionsfläche zu versehen. Insbesondere können Stiel- und/oder Kopfteile des Bandes mit einer glatten Rückenfläche aufgrund von Van-der-Waals-Kräften auch direkt verhaften.

Die Funktionsfläche zur Versorgung kann zumindest teilweise von mindestens einer Stielteile aufweisenden Funktionsfläche umgeben sein zur Ausbildung eines an einem die jeweilige Verletzung umgebenden Körperbereich, wie an Wundrandbereichen, anhaftenden Anhaftteils.

Auf diese Weise wird die Funktionsfläche dahingehend funktionalisiert, dass sie neben der Festlegung bzw. Fixierung des Medizinprodukts am Individuum zu dessen medizinischer Versorgung, beispielsweise durch Medikamente, mit beiträgt. Eine derartige Funktionsfläche ist beispielsweise mit Gold bedampft, mit einem sterilisierenden Medium, wie Silberpartikeln, versehen oder weist Fluiddurchlässe für ein während der Anlage des Medizinprodukts sukzessive an die Verletzung bzw. einen vorgegebenen Körperbereich abzugebendes Medikament auf.

Typischerweise ist die mindestens eine Funktionsfläche zur Versorgung Teil einer Versorgungsauflage, wie einer Wundauflage zur Versorgung einer Brandwunde. Die Versorgungsauflage kann am Träger des Medizinprodukts fixiert sein, alternativ kann die Funktionsfläche zur Versorgung unmittelbar am Träger ausgebildet sein. Hieraus ergibt sich der Vorteil, dass ein gebrauchsfertiges Medizinprodukt unter sterilen Bedingungen hergestellt und bevorratet werden kann.

Weiter ist es vorteilhaft, dass mindestens eine jeweils mindestens eine Funktionsfläche überdeckende, zum Gebrauch des Medizinprodukts abziehbare Schutzschicht, vorzugsweise mindestens eine Schutzfolie, vorgesehen ist. Durch die Schutzschicht wird eine unerwünschte Kontamination des Medizinprodukts sowie eine Anhaftung der Stielteile aufweisenden Funktionsfläche(n) an Drittflächen verhindert.

Die Erfindung betrifft weiter die Verwendung von gegenüber einer Funktionsfläche vorstehenden Stielteilen, deren freie stirnseitigen Enden derart ein Anhaftteil ausbilden, dass dieses zumindest teilweise an einem Individuum und/oder an einer weiteren Funktionsfläche überwiegend mittels Van-der-Waals-Kräften anhaftbar ist, an einem Medizinprodukt zur Versorgung eines mindestens eine, insbesondere großflächige, Verletzung, wie eine Brandwunde oder eine Hautabschürfung, aufweisenden Individuums.

In einer an Patienten mit Brandwunden an Fingern, Unterarmen und/oder Oberarmen durchgeführten Studie ist gezeigt worden, dass die Verwendung des erfindungsgemäßen Medizinprodukts die Dehnbarkeit, die Farbe, insbesondere die Rötung, die Hydration und die Schichtdicke der verbrannten Haut bzw. der Brandwunde deutlich gegenüber herkömmlichen Medizinprodukten verbessert hat. Insbesondere konnten verringerte Schmerzen bis hin zur Schmerzlosigkeit des einzelnen Patienten bei der Wiederabnahme bzw. Entfernung des Medizinprodukts vom Patienten erreicht werden.

In einer weiteren bevorzugten Ausführungsform des erfindungsgemäßen Medizinprodukts sind zumindest die Stielteile an der jeweiligen Funktionsfläche aus mindestens einem Kunststoffmaterial gebildet, wobei als Kunststoffmaterialien vorzugsweise anorganische und organische Elastomere, insbesondere Polyvinylsiloxan, additionsvernetzende Silikon-Elastomere, auch in der Form von Zwei-Komponenten-Systemen, sowie Acrylate eingesetzt sind. Ein Kunststoffmaterial bietet den Vorteil, dass beispielsweise ein flexibler Träger hergestellt werden kann, welcher vergleichbar einem Gewebepflaster oder einem Verband an stark konturierte Körperteile des zu versorgenden Individuums angelegt und an diesen fixiert werden kann.

Sehr gute Anhaftungsresultate ergeben sich, sofern mindestens 10.000, vorzugsweise jedoch 16.000, Stielteile pro cm$^2$ an der jeweiligen Funktionsfläche vorgesehen sind. Die Stielteile weisen bevorzugt jeweils einen Stielkörper mit der Form eines Rotationshyperboloids auf. Weiter können die Stielteile jeweils ein gegenüber einem Stielkörper verbreitertes Kopfteil aufweisen, wobei vorzugsweise die der Anhaftung zugeordneten Stirn- bzw. Oberseiten der verbreiterten Kopfteile im Wesentlichen plan oder leicht konvex ausgestaltet sind. Durch diese Maßnahmen werden die Anhaftung des durch die Stielteile ausgebildeten Anlageteils an einer entsprechenden Anlagefläche am Körper des Individuums sowie die Ablösung von der Anlagefläche weiter verbessert.

Zur Anhaftung der entsprechenden Funktionsfläche an der Anlagefläche genügt es, die Stielteile mit den freien verbreiterten Enden flächig an der Anlagefläche aufzusetzen. Vorzugsweise ist dabei vorgesehen, dass die Länge der Stielteile derart gewählt ist, dass diese über ihre freien Enden in einer gemeinsamen Ebene münden, da die Van-der-Waals-Kräfte nur über eine ausgesprochen kurze Distanz wirken. Vorteilhafterweise weist das jeweilige Stielteil und/oder der jeweilige Stielkörper eine Höhe von 50 $\mu$m bis 150 $\mu$m, vorzugsweise von etwa 90 $\mu$m, und einen Durchmesser von 10 $\mu$m bis 40 $\mu$m, vorzugsweise von etwa 30 $\mu$m, auf. Die gegenüber den Stielkörpern verbreiterten Kopfteile weisen vorteilhafterweise einen Durchmesser von 15 $\mu$m bis 70 $\mu$m, vorzugsweise von etwa 50 $\mu$m, auf.

Um zu vermeiden, dass die Stielteile von der zu kontaktierenden Anlagefläche wegknicken können, weisen diese eine hinreichende Eigensteifigkeit auf. Um ein gutes Ablöseverhalten sicherstellen zu können, kann vorgesehen sein, dass die verbreiterten Enden bzw. Kopfteile über eine entsprechende Durchmesserreduzierung im Übergangsbereich zum Stielkörper mit diesem verbunden sind. Auf diese Art und Weise entsteht an der Übergangsstelle eine Art Gelenk, so dass der vorzugsweise bandartige Träger mit den Stielkörpern an der entsprechenden Funktionsfläche bereits abgeschält wird und das dann noch anhaftende Kopfteil der Abschälbewegung im Sinne einer Abrollbewegung über das jeweilige Gelenk nachfolgt, was sich als besonders patientenschonend erwiesen hat.

Besonders günstig lässt sich die Herstellung des Medizinprodukts gestalten, wenn das jeweils verwendete Kunststoffmaterial thixotrop ist. Thixotropes Verhalten im Sinne der Erfindung soll dabei die Verringerung der Strukturstärke bedeuten während der Scherbelastungsphase und ihren mehr oder weniger schnellen, aber vollständigen Wiederaufbau während der nachfolgenden Ruhephase. Dieser Abbau/Wiederaufbau-Zyklus ist ein vollständig reversibler Vorgang und thixotropes Verhalten ist als zeitabhängiges Verhalten definierbar. Ferner haben sich Kunststoffmaterialien als günstig erwiesen, bei denen die mit einem Rotationsviskosimeter gemessene Viskosität von 7.000 bis 15.000 mPas reicht, vorzugsweise jedoch ein Wert von etwa 10.000 mPas bei einer Scherrate von $10 \frac{1}{\text{sec}}$ aufweist. Im Sinne einer sich selbst abreinigenden Oberfläche hat es sich darüber hinaus als günstig erwiesen, Kunststoffmaterialien zu verwenden, deren Kontaktwinkel aufgrund ihrer Oberflächenenergie für die Benetzung mit Wasser mindestens einen Wert von größer 60 grd aufweist.

Weitere Vorteile und Merkmale der Erfindung ergeben sich aus den Figuren und der nachfolgenden Beschreibung der Zeichnung. Die vorstehend genannten und die weiter angeführten Merkmale können in beliebigen Kombinationen an einem erfindungsgemäßen Medizinprodukt bzw. bei der erfindungsgemäßen Verwendung realisiert sein. Die in den Figuren gezeigten Merkmale sind rein schematisch und nicht maßstäblich zu verstehen. Es zeigt:

Fig. 1a und 1b     jeweils ein beispielhaftes Medizinprodukt gemäß der Erfindung;

Fig. 2     einen stark vergrößerten Ausschnitt des Medizinprodukts aus Fig. 1 a zur Veranschaulichung der Stielteile an einer Funktionsfläche des Medizinprodukts;

Fig. 3     einen Längsschnitt durch einen Formhohlraum zur Ausbildung eines in Fig. 2 gezeigten Stielteils nebst Kopfteil;

Fig. 4     eine Seitenansicht auf eine Vorrichtung zur Herstellung von Trägermaterial für ein erfindungsgemäßes Medizinprodukt, mit einer Formhohlräume nach Fig. 3 aufweisenden Formwalze;

Fig. 5a      ein weiteres beispielhaftes Medizinprodukt gemäß der Erfindung;

Fig. 5b      einen Schnitt durch das Medizinprodukt aus Fig. 5a in Gebrauchsstellung;

Fig. 6a      ein weiteres beispielhaftes Medizinprodukt gemäß der Erfindung; und

Fig. 6b      einen Schnitt durch das Medizinprodukt aus Fig. 6a mit einer zum Gebrauch des Medizinprodukts abziehbaren Schutzfolie.

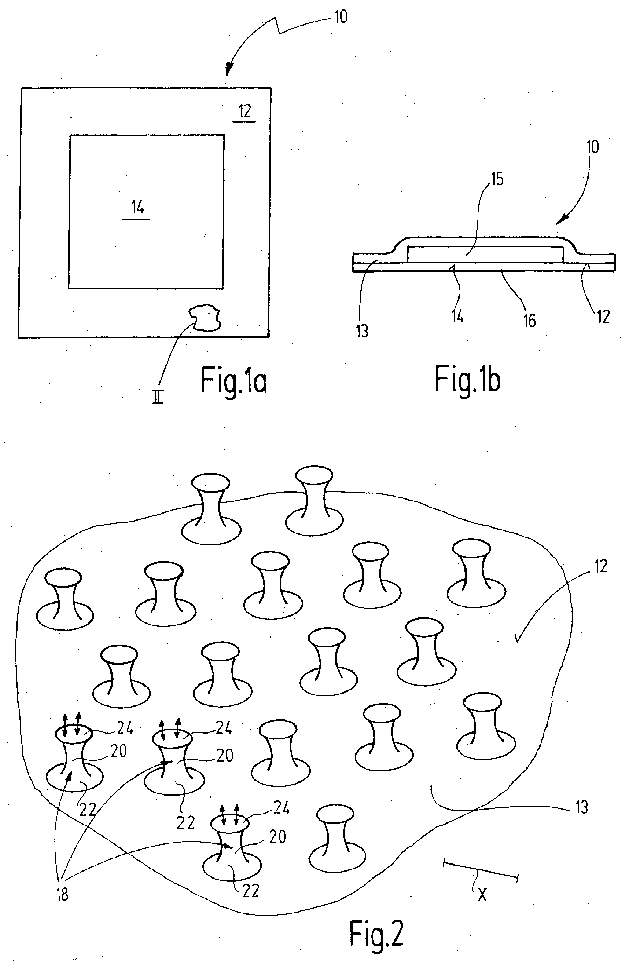

Fig. 1 a zeigt in Draufsicht ein Medizinprodukt 10, welches in der Art eines Pflasters ausgebildet ist und auf der im Gebrauch dem zu versorgenden Individuum, wie einem Patienten, zugewandten bzw. an diesem anliegenden und anhaftenden Seite eine Funktionsfläche 12 zur Anhaftung und eine weitere Funktionsfläche 14 zur Versorgung der abzudeckenden Verletzung aufweist. Die Funktionsflächen 12, 14 sind jeweils rechteckförmig, im gezeigten Beispiel quadratisch, ausgebildet und derart zueinander angeordnet, dass die eine Funktionsfläche 12 die weitere Funktionsfläche 14 umgibt und in Randbereichen des Medizinprodukts 10 eine Anhaftung am Individuum, wie an einer Anlagefläche am Körper des Patienten, ermöglicht.

Der in Fig. 1b gezeigte Schnitt durch das Medizinprodukt 10 aus Fig. 1a zeigt, dass die eine Funktionsfläche 12 an einem Träger 13 und die weitere Funktionsfläche 14 an einer Versorgungsauflage 15 ausgebildet sind. Die Versorgungsauflage 15 kann beispielsweise zur Aufnahme von Wundsekret, d.h. saugend, ausgebildet sein. Das Medizinprodukt 10 als Ganzes ist sandwichartig mit dem Träger 13 aus einem Kunststoffmaterial, der Versorgungsauflage 15 und einer die Funktionsflächen 12, 14 überdeckenden Schutzschicht in der Art einer Schutzfolie 16 aufgebaut. Die Schutzfolie 16 ist aus Papier und/oder Kunststoff hergestellt und wird zum Gebrauch des Medizinprodukts 10 von den Funktionsflächen 12, 14 abgenommen. Zweckmäßigerweise ist die Versorgungsauflage 15 mit dem Träger 13 fest verbunden, beispielsweise verklebt, es ist jedoch auch denkbar, die Verbindungsfläche zwischen dem Träger 13 und der Versorgungsauflage 15 entsprechend der Funktionsfläche 12, d.h. mit Stielteilen, zu versehen, so dass beim Verbandswechsel lediglich die Versorgungsauflage 15 auszutauschen ist und der Träger 13 wiederverwendet werden könnte.

Die Abmaße des Medizinprodukts 10 sowie des Trägers 13 und entsprechend der Versorgungsauflage 15 sind vielfältig, beispielsweise rund, elliptisch, quadratisch, rechteckförmig, dreieckig, etc. wählbar. Je nach gewünschtem Anwendungsbereich und an die Anhaftkraft gestellten Anforderungen werden die Anzahl und die Größe der Funktionsflächen 12, 14 gewählt. Die im Randbereich des Medizinprodukts 10 vorgesehene Funktionsfläche 12 zeigt eine gute Produktanhaftung am Körper, typischerweise an der Haut, des Patienten und schützt auf diese Weise die Versorgungsauflage 15 beispielsweise vor dem Eindringen von Kontaminationen von außen sowie vor dem Austrocknen. Die Mikrostruktur der Funktionsfläche 12 ist in Fig. 2 dargestellt. Die an der Funktionsfläche 12 des Trägers 13 ausgebildeten Stielteile 18 stehen jeweils gegenüber der Funktionsfläche 12 vor und weisen einen Stielkörper 20, ein den Stielkörper 20 mit der Funktionsfläche 12 verbindendes Fußteil 22 und ein vom Fußteil 22 abgewandtes, gegenüber dem Stielkörper 20 verbreitertes Kopfteil 24 auf. Die Stirnbzw. Oberseite des jeweiligen Kopfteils 24 stellt, wie in Fig. 2 mit Doppelpfeilen dargestellt, die effektive Fläche zur Ausbildung von Van-der-Waals-Kräften mit einer in Fig. 2 nicht gezeigten zugeordneten Anlagefläche dar, beispielsweise auch in Form von Haut- oder einer Wundfläche gebildet.

Um die Größenverhältnisse der Stielteile 18 zu verdeutlichen, ist in der Fig. 2 mit X eine Länge bezeichnet, die der Größe von etwa 100 $\mu$m entspricht. Von der Funktionsfläche 12 des Trägers 13 aus gerechnet bis zum Abschluss des Stielteils 18 an der planen Oberseite bzw. Stirnseite des Kopfteils 24 weist jedes Stielteil 18 eine Höhe von etwa 100 $\mu$m auf, was dem Größenmaßstab X nach der Fig. 2 entspricht. Die planen Oberseiten der Kopfteile 24 haben jeweils einen Durchmesser von etwa 50 $\mu$m und verringern sich in Richtung zum oberen Ende des jeweiligen Stielkörpers 20 auf eine Größe von etwa 30 $\mu$m. Insoweit ist zwischen dem jeweiligen Kopfteil 24 und dem jeweiligen Stielkörper 20 an der Stelle des Übergangs eine Art Hinterschnitt gebildet. Die Höhe des jeweiligen Kopfteils 24 beträgt etwa 10 $\mu$m und die Größe des radialen Überstandes vom Kopfteil 24 zum oberen Ende des zugehörigen Stielkörper 20 beträgt ca. 10 $\mu$m. Die Abstände zwischen den Begrenzungen von einander benachbart gegenüberliegenden Kopfteilen 24 betragen 30 $\mu$m bis 40 $\mu$m. Der Durchmesser des jeweiligen Stielkörpers 20 liegt bei etwa 20 $\mu$m bis 35 $\mu$m.

Die dahingehenden Größenverhältnisse sind nur beispielhaft und können im genannten Größenrahmen geändert werden, wobei jedenfalls sichergestellt sein muss, dass das jeweilige Kopfteil 24 eine plane oder geringfügig konvexe Stirn- bzw. Oberseite aufweist, die die Wirkung von Van-der-Waals-Kräften ermöglicht, sofern die Funktionsfläche 12 mit einer Anlagefläche beliebiger Art in Berührung kommt. Bei dem hier großtechnisch herstellbaren Anlageteil sind aufgrund der Nanogestaltung der Stielteile 18 diese mit dem bloßen Auge nicht mehr zu erkennen, und es ist überraschend, dass aufgrund des Aufbaus aus Stielteilen 18 eine sehr sichere lösbare Verhaftung über die Van-der-Waals-Kräfte erfolgt.

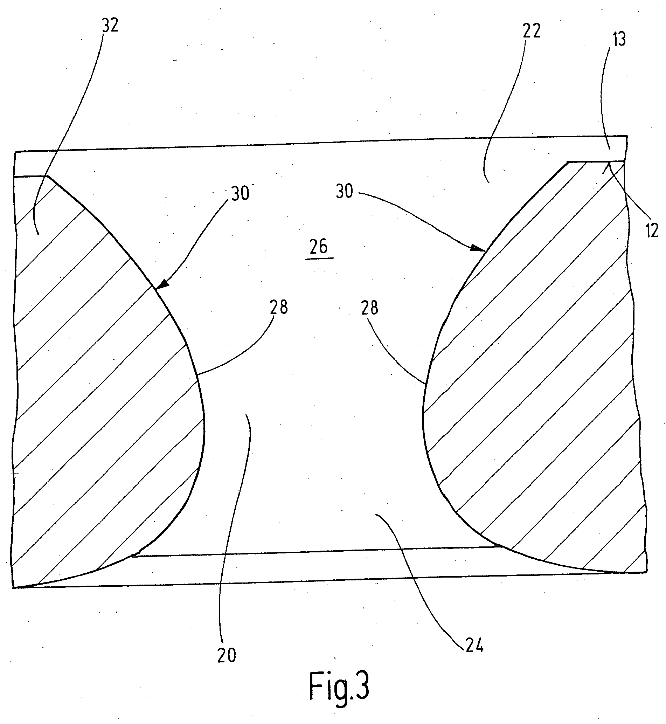

Sowohl die Kopfteile 24 als auch die Stielkörper 20 können im Querschnitt eckig, insbesondere mit einer hexagonalen Querschnittsform, versehen sein, und das Aspektverhältnis eines jeden Stielteils 18 liegt vorzugsweise zwischen 1:3 und 1:5. Die Form der einzelnen Stielteile 18 lässt sich Fig. 3 entnehmen, welche im Längsschnitt einen Formhohlraum 26 zur Ausbildung eines Stielteils 18 mit einem Fußteil 22, einem Stielkörper 20 und einem gegenüber dem Stielkörper 20 verbreiterten Kopfteil 24 zeigt. Fig. 3 gibt einen Längsschnitt wieder eines Formhohlraums 26, wobei die im Längsschnitt gegenüberliegend gezeigte Begrenzungswand 30 einen konvexen Bahnverlauf aufweist. Die Krümmung des Bahnverlaufs ist in Richtung des zu formenden Kopfteils 24 stärker ausgeführt als in Richtung des Fußteils 22, über welches der Stielkörper 20 mit dem Träger 13 verbunden ist. Das Stielteil 18 steht gegenüber der am Träger 13 ausgebildeten Funktionsfläche 12 vor.

Als besonders vorteilhaft hat es sich erwiesen, wenn man von der Längsrichtung des Stielkörpers 20 aus in Richtung des Kopfteils 24 gesehen den Bahnverlauf der Begrenzungswand 30 mit seiner stärksten Krümmung oberhalb der Mitte, vorzugsweise im oberen Drittel, beginnend vorsieht. Zur Ausbildung eines Stielteils 18 wird Kunststoffmaterial in den Formhohlraum 26 gedrängt, kommt dort zur Ruhe und erstarrt bzw. härtet dort zumindest teilweise aus. Die mittlere Einschnürung 28, bedingt durch die Formgebung des Rotationshyperboloids, lässt sich weiter dergestalt fortführen, dass bei einer weiter verengten Stelle eine Art Gelenk zwischen dem Stielkörper 20 und dem Kopfteil 24 entsteht. Ein derartiges Gelenk ist günstig für das Van-der-Waals-Anhaftungssystem.

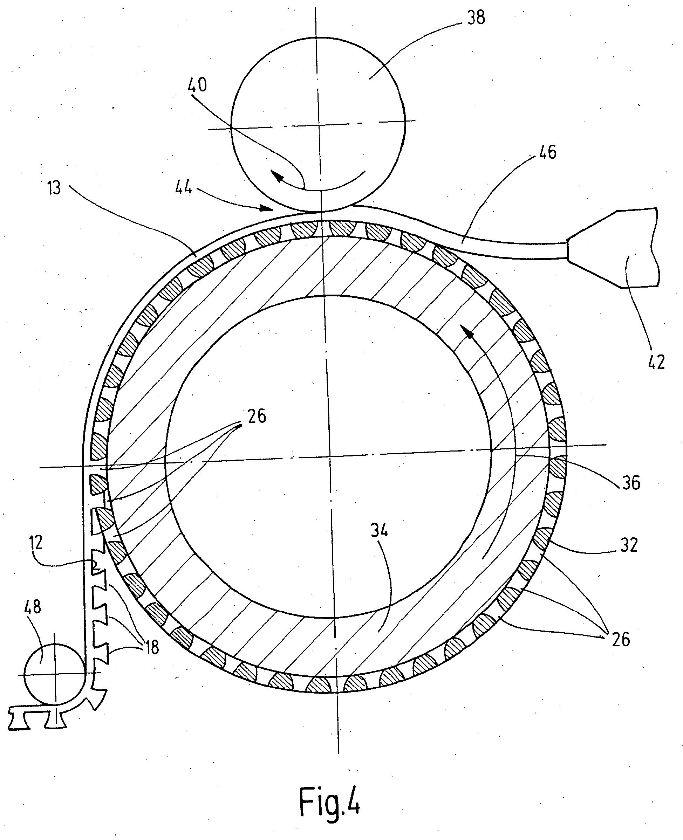

Fig. 4 zeigt in schematischer Darstellung Teile einer Vorrichtung zur Herstellung eines Trägermaterials 46 für ein erfindungsgemäßes Medizinprodukt bzw. die erfindungsgemäße Verwendung. An einer Formwalze 34 ist ein Sieb 32 mit einer Vielzahl von Formhohlräumen 26, die jeweils die in Fig. 3 gezeigte Ausgestaltung aufweisen, außenumfangsseitig angeordnet.

Die Formwalze 34 ist in einer ersten Drehrichtung 36, gegen den Uhrzeigersinn, dreh- bzw. rotierbar. Weiter ist eine Druckwalze 38 vorgesehen, welche in einer zweiten Drehrichtung 40, im Uhrzeigersinn, dreh- bzw. rotierbar ist und in Bezug zur Formwalze 34 derart angeordnet ist, dass ein Formspalt 44 ausgebildet ist. Als Zuführeinrichtung für plastisches, flüssiges und/oder thixotropes Kunststoffmaterial ist ein Düsenkopf 42 vorgesehen. Vom Düsenkopf 42 wird Trägermaterial 46 zum Förderspalt 44 geführt und durch den von der Druckwalze 38 aufgebrachten Druck auf der der Formwalze 34 zugewandten Seite in die Formhohlräume 26 des Siebes 32 gedrückt. Die der Formwalze 34 zugeordnete Seite des Trägermaterials 46 gibt die Funktionsfläche 12 am Träger 13 vor. Auf dem Weg vom Formspalt 44 zu einer Umlenkwalze 48 härtet das in die Formhohlräume 26 eingedrückte Kunststoffmaterial zumindest teilweise aus und die entsprechend geformten, einstückig am Träger 13 ausgebildeten Stielteile 18 werden aus den Formhohlräumen 26 geführt.

Mittels der in Fig. 4 gezeigten Vorrichtung kann Trägermaterial 46 bzw. der Träger 13 mit gegenüber der Funktionsfläche 12 vorstehenden Stielteilen 18 als Endlosmaterial hergestellt werden und in einer sich an die Umlenkwalze 48 anschließenden, in Fig. 4 nicht gezeigten Schneideinrichtung entsprechend der gewählten Größe zugeschnitten werden. Das über den Düsenkopf 42 zugeführte Trägermaterial 46 kann eine Art Kunststoffband sein. Die Formhohlräume 26 am Sieb 32 der Formwalze 34 sind entsprechend der Ausgestaltung und Anordnung der Stielteile 18 am Träger 13 angeordnet. Typischerweise sind die Formhohlräume 26 am Außenumfang der Formwalze 34 regelmäßig verteilt mit vorzugsweise mehr als 10.000 Formhohlräumen 26 pro cm$^2$ auf dem Sieb 32.

Als besonders vorteilhaft in der medizinischen Applikation hat es sich jedoch erwiesen, als Medizinprodukt ein Produkt gemäß der Darstellung nach der Fig. 2 einzusetzen, und zwar dergestalt, dass die jeweiligen Stielkörper 20 mit ihren vorzugsweise verbreiterten Kopfteilen 24 in direkte Anlage mit der Verletzung, wie einem Wundbereich bzw. Körper- und Hautteilen des Patienten, kommen, wobei die Funktionsfläche 12 den Wundbereich vollständig abdeckt. Sofern der Träger 13 mit der Funktionsfläche 12 in der Art eines Wickelbandes ausgebildet ist, lässt sich auch der jeweilige Wundbereich am Körper des Patienten umwickeln, so dass eine vollständige Abschirmung des Wundbereiches erreicht ist und aufgrund der mikroreplikativen Anhaftung der Kopfteile 24 im Wundbereich lässt sich das Trägerband bzw. der Träger 13 schmerzfrei wieder von der Wundstelle abnehmen und gegen ein Neuband eintauschen.

Aufgrund des ausgewählten Kunststoffmaterials ist das in Fig. 2 ausschnittsweise dargestellte Gesamt- bzw. Medizinprodukt 10 ähnlich von seinen chemischen und physikalischen Eigenschaften her einer Hautstruktur nachempfunden, so dass eine Art Kunsthaut entsteht, die die fehlende menschliche Haut oder Tierstruktur zumindest für eine längere Verweildauer ersetzen kann. Insbesondere Infektionen im Wundbereich sind durch die erfindungsgemäße Abdeckung mit Sicherheit vermieden. Anstelle von verbreiterten Kopfteilen 24 lassen sich zylindrisch ausgebildete Stielteile an ihrem jeweiligen Ende auch in Einzelfilamente (nicht dargestellt) aufsplitten für eine Anhaftung der Einzelfilamente mittels Van-der-Waals-Kräften.

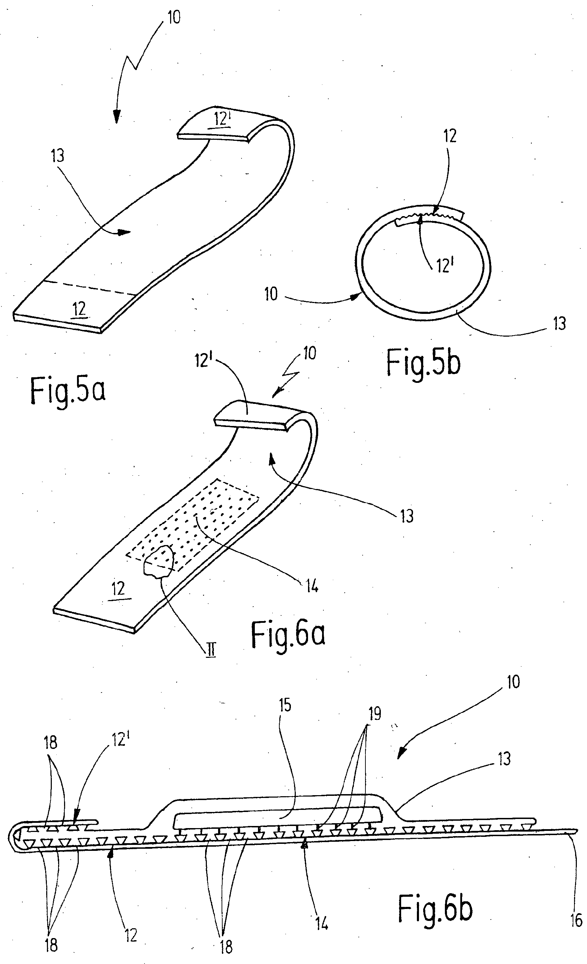

Fig. 5a zeigt in Draufsicht ein weiteres Medizinprodukt 10, welches in der Art eines Verbandes als bandartiges Flächengebilde ausgebildet ist. Ein bandartiger Träger 13 weist an seinen beiden Enden jeweils eine Funktionsfläche 12, 12' auf, von denen die eine Funktionsfläche 12 an der im Gebrauch dem zu versorgenden Patienten zugewandten bzw. an diesem anliegenden Seite des Trägers 13 ausgebildet ist und die andere Funktionsfläche 12' auf der im Gebrauch vom zu versorgenden Patienten abgewandten Seite des Trägers 13 ausgebildet ist. In der in Fig. 5b gezeigten Gebrauchsstellung des Medizinprodukts 10 ist der bandartige Träger 13 in radialer Richtung um ein in Fig. 5b nicht gezeigtes Körperteil eines Patienten derart gewickelt, dass sich die Endbereiche des Trägers 13 überlappen, so dass die beiden Funktionsflächen 12, 12' in Aneinanderlage gebracht werden können. Durch die Aneinanderlage der Funktionsflächen 12, 12' wird, wie in Fig. 5b gezeigt, ein wieder lösbarer Verschluss an den Enden des Trägers 13 ausgebildet und das Medizinprodukt 10 durch eine radiale Anpresskraft am Patienten fixiert.

Es versteht sich, dass die in der Gebrauchsstellung des Medizinprodukts 10 radial innenliegende Funktionsfläche 12 vollflächig an der entsprechenden Seite des Trägers 13 ausgebildet sein kann. Des Weiteren kann die in Gebrauchsstellung radial außenliegende weitere Funktionsfläche 12' vollflächig an der entsprechenden Seite des Trägers 13 ausgebildet sein, wodurch das Medizinprodukt 10 flexibel an unterschiedlich großen Körperteilen des Patienten angelegt und dort fixiert werden kann. Des Weiteren sind bei einer mehrfachen bzw. mehrlagigen Umwicklung des jeweiligen Körperteils mit zumindest teilweise übereinanderliegenden Lagen des Trägers 13 diese in ihrer jeweiligen Lage fixierbar. In Fig. 5b ist eine Einfachwicklung des Trägers 13 um einen Körperteil des Patienten gezeigt, es sind jedoch auch Mehrfachwicklungen möglich.

Das in Fig. 6a in perspektivischer Ansicht gezeigte weitere Medizinprodukt 10 unterscheidet sich von dem in Fig. 5a gezeigten Ausführungsbeispiel dadurch, dass die eine Funktionsfläche 12 an der im Gebrauch dem Patienten zugewandten Seite des Trägers 13 vollflächig ausgebildet ist. Weiter ist in einem mittleren Bereich der entsprechenden Seite des Trägers 13 eine weitere im Wesentlichen rechteckförmige Funktionsfläche 14 zur Wundversorgung ausgebildet. Sowohl die eine Funktionsfläche 12 als auch die weitere Funktionsfläche 14 weisen die in Fig. 2 gezeigte Oberflächenstruktur mit Stiel- und Kopfteilen (in Fig. 6a nicht bezeichnet) zur Ausbildung einer Anhaftung überwiegend mittels Van-der-Waals-Kräften auf.

Im in Fig. 6b gezeigten Schnitt durch das Medizinprodukt 10 sind die Stielteile 18 an den Funktionsflächen 12, 12', 14 übergroß dargestellt. In den Träger 13 ist eine weitere Funktionsauflage 15 zur Wundversorgung eingebettet, welche beispielsweise ein an einem Wundbereich abzugebendes Medikament enthält. Über Fluiddurchlässe 19 im Träger 13 ist die Funktionsfläche 14 im Gebrauch an den Patienten anlegbar und das in der Versorgungsauflage 15 enthaltene Medikament entsprechend abgebbar. Die vom die Versorgungsauflage 15 aufnehmenden Innenraum des Trägers 13 zur Funktionsfläche 14 führenden Fluiddurchlässe 19 bilden, wie in Fig. 6a gezeigt, eine Art Perforation des Trägers 13 im Bereich der Funktionsfläche 14 aus.

Durch eine Schutzfolie 16 sind die Funktionsflächen 12, 12', 14 vor dem Gebrauch des Medizinprodukts 10, genauer vor einer Anlage und Fixierung am Patienten, geschützt. Im gezeigten Ausführungsbeispiel ist eine Schutzfolie 16 für die Funktionsflächen 12, 12', 14 an beiden Seiten, Unter- und Oberseite, des Trägers 13 vorgesehen, es ist jedoch auch denkbar, zwei voneinander getrennte Schutzfolien an beiden Seiten des Trägers 13 vorzusehen. Am der weiteren Funktionsfläche 12' gegenüberliegenden Ende weist die Schutzfolie 16 gegenüber dem Träger 13 einen Überstand auf, wodurch das Greifen bzw. Fassen der Schutzfolie 16 zum Ablösen vom Träger 13 bzw. von den Funktionsflächen 12, 12' durch eine Bedienperson, insbesondere durch medizinisches Fachpersonal, erleichtert ist.

Im einfachsten Fall ist die medizinische Funktionsfläche durch ein Trägerband gebildet, auf dessen einer Seite die Stielteile 18 dann vorstehen. In Abhängigkeit der Wundsituation kann dann die Fläche mit den Stielteilen 18 auf die Wunde aufgelegt werden oder die entgegengesetzt liegende glatte Rückseite des Trägerbandes. Wird das Trägerband um ein Körperteil des Individuums herumgewickelt, können die vorstehenden Stielteile 18 die Verhaftung an der glatten Rückseite des Trägerbandes wahrnehmen und es ist für das jeweilige Individuum sehr angenehm, dass der Grad der Wicklungskraft sehr einfach mit dem Band einstellbar ist, so dass sich von sehr lockeren Wicklungen bis zu sehr festen Wicklungen je nach Bedarfsfall die Wicklungskräfte vorgeben lassen.

Sofern von der Anhaftung der freien Stielteilenden mittels Van-der-Waals-Kräften die Rede ist, meint dies, dass das freie stirnseitige Ende des jeweiligen Stielteils die dahingehende Anhaftung wahrnehmen kann; es besteht aber auch die Möglichkeit, das freie Ende des zylindrisch ausgebildeten Stielteils in eine Vielzahl von Einzelfilamenten aufzuteilen, um dergestalt die Anhaftung zu ermöglichen. Wie bereits dargelegt, kann aber auch das freie Kopfende des Stielteils vom Durchmesser des Stielteils her verbreitert werden und beliebige Formen einnehmen, wie Vielecke, auch in regelmä-ßiger Form, zylindrische oder elliptische Formen sowie Sonderformen, wie Kleeblattstrukturen oder chrysanthemenartige Ausgestaltungen. Die genannte Folienbahn kann auch mehrlagig aufgebaut sein und sowohl auf der Seite mit den Stiel- und Kopfteilen eine Beschichtung aufweisen als auch auf der Rückseite.

Sofern mit der vorliegenden Anmeldung Individuen angesprochen sind, betrifft dies Patienten, aber auch Tiere, und aufgrund des guten Anhaftungsverhaltens des Wundbandes steht zu erwarten, dass dieses sogar im Bereich der Aquaristik für Fische vollumfänglich einsetzbar ist. Ferner haben neuere Studien gezeigt, dass das Trägerband nahezu in der Art eines Hautersatzes dienen kann und im Hinblick auf die dünne Folienausgestaltung kann das Band auch mit Haut- und Gewebeteilen randseitig vernäht oder eingenäht werden, so dass weitere Anwendungen im operativen Bereich möglich sind einschließlich im Bereich der Augenheilkunde und sonstigen plastischen Chirurgie. Ferner lassen sich auch interne Wund- und Operationsbereiche mit dem bandartigen Folienmaterial vollflächig abdecken und auch Blutungen wirksam beherrschen.

**Patentansprüche**

1. Medizinprodukt (10) zur Versorgung eines mindestens eine, insbesondere großflächige, Verletzung, wie eine Brandwunde oder eine Hautabschürfung, aufweisenden Individuums, umfassend mindestens eine Funktionsfläche (12, 12', 14) zur zumindest teilweisen Abdeckung der jeweiligen Verletzung und/oder zur Festlegung des Medizinprodukts (10) am Individuum, wobei mindestens eine Funktionsfläche (12, 12', 14) von ihr vorstehende Stielteile (18) aufweist, deren freien stirnseitigen Enden derart ein Anhaftteil ausbilden, dass dieses zumindest teilweise am Individuum und/oder an einer weiteren Funktionsfläche (12') überwiegend mittels Van-der-Waals-Kräften anhaftbar ist, wobei das Medizinprodukt (10) mindestens eine Funktionsfläche (14) zur Versorgung und/oder zur Anlage an der jeweiligen Verletzung des Individuums, wie an einem Wundbereich, aufweist, **dadurch gekennzeichnet, dass** die Funktionsfläche (14) zur Versorgung von einer fluiddurchlässigen, vorzugsweise perforierten, Funktionslage überdeckt ist und dass die Funktionslage an ihrer der jeweiligen Verletzung zugewandten Seite eine Funktionsfläche (14) mit Stielteilen (18) zur Ausbildung eines an der jeweiligen Verletzung anhaftenden Anhaftteils aufweist.

2. Medizinprodukt nach Anspruch 1, **dadurch gekennzeichnet, dass** das Medizinprodukt (10) einen Träger (13) aufweist, welcher mindestens eine Funktionsfläche (12, 12') aufweist und als vorzugsweise bandartiges Flächengebilde ausgebildet ist.

3. Medizinprodukt nach Anspruch 1 oder 2, **dadurch gekennzeichnet, dass** die Funktionsfläche (14) zur Versorgung zumindest teilweise von mindestens einer Stielteile (18) aufweisenden Funktionsfläche (12) umgeben ist zur Ausbildung eines an einem die jeweilige Verletzung umgebenden Körperbereich, wie an Wundrandbereichen, anhaftenden Anhaftteils.

4. Medizinprodukt nach einem der vorhergehenden Ansprüche, **dadurch gekennzeichnet, dass** die mindestens eine Funktionsfläche (14) zur Versorgung Teil einer Versorgungsauflage (15), wie einer Wundauflage zur Versorgung einer Brandwunde, ist.

5. Medizinprodukt nach einem der vorhergehenden Ansprüche, **dadurch gekennzeichnet, dass** mindestens eine jeweils mindestens eine Funktionsfläche (12, 12', 14) überdeckende, zum Gebrauch des Medizinprodukts (10) abziehbare Schutzschicht, vorzugsweise mindestens eine Schutzfolie (16), vorgesehen ist.

6. Medizinprodukt nach einem der vorhergehenden Ansprüche, **dadurch gekennzeichnet, dass** zumindest die Stielteile (18) an der jeweiligen Funktionsfläche (12, 12') aus mindestens einem Kunststoffmaterial gebildet sind, wobei als Kunststoffmaterialien vorzugsweise anorganische und organische Elastomere, insbesondere Polyvinylsiloxan, additionsvernetzende Silikon-Elastomere, auch in der Form von Zwei-Komponenten-Systemen, sowie Acrylate eingesetzt sind.

7. Medizinprodukt nach Anspruch 6, **dadurch gekennzeichnet, dass** das jeweils verwendete Kunststoffmaterial thixotrop ist und eine mit einem Rotationsviskosimeter gemessene Viskosität von 7.000 bis 15.000 mPas, vorzugsweise jedoch von etwa 10.000 mPas, bei einer Scherrate von $10 \, \frac{1}{\text{sec}}$ aufweist.

8. Medizinprodukt nach einem der vorhergehenden Ansprüche, **dadurch gekennzeichnet, dass** mindestens 10.000, vorzugsweise jedoch 16.000, Stielteile (18) pro cm$^2$ an der jeweiligen Funktionsfläche (12, 12') vorgesehen sind.

9. Medizinprodukt nach einem der vorhergehenden Ansprüche, **dadurch gekennzeichnet, dass** die Stielteile (18) an ihren freien Enden spatulaartig aufgetrennt sind.

10. Medizinprodukt nach einem der Ansprüche 1 bis 10, **dadurch gekennzeichnet, dass** die Stielteile (18) jeweils einen Stielkörper (20) mit der Form eines Rotationshyperboloids aufweisen.

11. Medizinprodukt nach einem der Ansprüche 1 bis 10, 12, **dadurch gekennzeichnet, dass** Stielteile (18) jeweils ein gegenüber einem Stielkörper (20) verbreitertes Kopfteil (24) aufweisen, wobei vorzugsweise die der Anhaftung zugeordneten Oberseiten der verbreiterten Kopfteile (24) im Wesentlichen plan oder leicht konvex ausgestaltet sind.

12. Medizinprodukt nach einem der vorhergehenden Ansprüche, **dadurch gekennzeichnet, dass** das jeweilige Stielteil (18) und/oder der jeweilige Stielkörper (20) eine Höhe von 50 $\mu$m bis 150 $\mu$m, vorzugsweise von etwa 90 $\mu$m, und einen Durchmesser von 10 $\mu$m bis 40 $\mu$m, vorzugsweise von etwa 30 $\mu$m, aufweisen.

13. Medizinprodukt nach Anspruch 13 oder 14, **dadurch gekennzeichnet, dass** die gegenüber den Stielkörpern (20) verbreiterten Kopfteile (24) einen Durch-

messer von 15 $\mu$m bis 70 $\mu$m, vorzugsweise von etwa 50 $\mu$m, aufweisen.

## Claims

1. A medicinal product (10) for the care of an individual having at least one injury such as a burn wound or a skin abrasion, in particular with a large surface area, comprising at least one functional surface (12, 12', 14) for covering the respective injury at least partially and/or for fixing the medicinal product (10) on the individual, at least one functional surface (12, 12', 14) having stem parts (18) projecting from it, the free front-side ends of which form an adhesion part such that the latter can be adhered at least partially on the individual and/or on another functional surface (12'), primarily by means of van der Waals forces, the medicinal product (10) having at least one functional surface (14) for caring for and/or for placing on the respective injury of the individual, such as on a wound region, **characterised in that** the functional care surface (14) is covered by a fluid-permeable, preferably perforated functional layer and that the functional layer has on its side facing the respective injury a functional surface (14) with stem parts (18) for forming an adhesion part adhering on the respective injury.

2. The medicinal product according to Claim 1, **characterised in that** the medicinal product (10) comprises a carrier (13) that has at least one functional surface (12, 12') and is formed as a preferably band-like surface structure.

3. The medicinal product according to Claim 1 or 2, **characterised in that** the functional care surface (14) is surrounded at least partially by at least one functional surface (12) comprising stem parts (18) for forming an adhesion part that adheres on a body region, such as on edge regions of the wound, surrounding the respective injury.

4. The medicinal product according to any of the preceding claims, **characterised in that** the at least one functional care surface (14) is part of a care coating (15) such as a wound coating for the care of a burn wound.

5. The medicinal product according to any of the preceding claims, **characterised in that** at least one protective layer, preferably at least one protective film (16), is provided that respectively covers at least one functional surface (12, 12', 14) and can be pulled off in order to use the medicinal product (10).

6. The medicinal product according to any of the preceding claims, **characterised in that** at least the stem parts (18) on the respective functional surface (12, 12') are formed from at least one plastic material, inorganic and organic elastomers, in particular polyvinyl siloxane, addition-linking silicone elastomers, also in the form of two-component systems, as well as acrylates preferably being used as plastic materials.

7. The medicinal product according to Claim 6, **characterised in that** the respectively used plastic material is thixotropic and has a viscosity measured with a rotary viscosity meter of from 7,000 to 15,000 mPas, but preferably of approximately 10,000 mPas, at a shearing rate of 10 1/sec.

8. The medicinal product according to any of the preceding claims, **characterised in that** at least 10,000, but preferably 16,000 stem parts (18) are provided per cm2 on the respective functional surface (12, 12').

9. The medicinal product according to any of the preceding claims, **characterised in that** the stem parts (18) are separated in the manner of a spatula on their free ends.

10. The medicinal product according to any of Claims 1 to 10, **characterised in that** the stem parts (18) each have a stem body (20) in the form of a rotational hyperboloid.

11. The medicinal product according to any of Claims 1 to 10, **characterised in that** the stem parts (10) each have a head part (24) that is widened in comparison to a stem body (20), the top sides of the widened head parts (24) associated with the adhesion preferably being designed to be substantially planar or slightly convex.

12. The medicinal product according to any of the preceding claims, **characterised in that** the respective stem part (18) and/or the respective stem body (20) has a height of 50 $\mu$m to 150 $\mu$m, preferably of approximately 90 $\mu$m, and a diameter of 10 $\mu$m to 40 $\mu$m, preferably of approximately 30 $\mu$m.

13. The medicinal product according to Claim 13 or 14, **characterised in that** the head parts (24), widened in comparison to the stem parts (20), have a diameter of 15 $\mu$m to 70 $\mu$m, preferably of approximately 50 $\mu$m.

## Revendications

1. Produit (10) médical pour soigner une personne ayant au moins une blessure, notamment de grande surface, comme une brûlure ou une excoriation, comprenant au moins une surface (12, 12', 14) fonctionnelle pour recouvrir au moins en partie la blessure et/ou pour maintenir le produit (10) médical sur la personne, dans lequel au moins une surface (12, 12', 14) fonctionnelle a des parties (18) de tige, qui en font saillie et dont les extrémités libres du côté frontal forment une partie d'adhérence, de manière à ce que celle-ci puisse, au moins en partie, adhérer, d'une manière prépondérante aux moyens de force de Van-der-Waals, à la personne et/ou à une autre surface (12') fonctionnelle, le produit (10) médical ayant au moins une surface (14) fonctionnelle pour soigner et/ou pour s'appliquer à la blessure de la personne, comme une zone de brûlure, **caractérisé en ce que** la surface (14) fonctionnelle pour soigner est recouverte d'une couche fonctionnelle perméable au fluide, de préférence perforée, et **en ce que** la couche fonctionnelle a, sur sa face tournée vers la blessure, une surface (14) fonctionnelle ayant des parties (18) de tige pour former une partie d'adhérence adhérant à la blessure.

**2.** Produit médical suivant la revendication 1, **caractérisé en ce que** le produit (10) médical a un support (13), qui a au moins une surface (12, 12') fonctionnelle et qui est constitué sous la forme d'une étoffe, de préférence de type en bande.

**3.** Produit médical suivant la revendication 1 ou 2, **caractérisé en ce que** la surface (14) fonctionnelle pour soigner est entourée, au moins en partie, d'au moins une surface (12) fonctionnelle ayant des parties (18) de tige pour constituer une partie d'adhérence adhérant à une zone du corps entourant la blessure, comme des zones au bord d'une plaie.

**4.** Produit médical suivant l'une des revendications précédentes, **caractérisé en ce que** la au moins une surface (14) fonctionnelle pour soigner fait partie d'une couche (15) de soin, comme une couche de plaie pour soigner une brûlure.

**5.** Produit médical suivant l'une des revendications précédentes, **caractérisé en ce qu'**il est prévu au moins une couche de protection, de préférence au moins une feuille (16) de protection, recouvrant au moins une surface (12, 12', 14) fonctionnelle et pouvant être retirée pour utiliser le produit (10) médical.

**6.** Produit médical suivant l'une des revendications précédentes, **caractérisé en ce qu'**au moins les parties (18) de tige, sur la surface (12, 12') fonctionnelle, sont en au moins une matière plastique, dans lequel il est utilisé comme matière plastique de préférence des élastomères minéraux et organiques, notamment du polyvinylsiloxane, des élastomères de silicone réticulés par addition, également sous la forme de système à deux constituants, ainsi que des acrylates.

**7.** Produit médical suivant la revendication 6, **caractérisé en ce que** la matière plastique utilisée est thixotrope et a une viscosité mesurée par un viscosimètre tournant de 7.000 à 15.000 mPas, mais de préférence d'environ 10.000 mPas, à une vitesse de cisaillement de $10 \; \frac{1}{sec}$.

**8.** Produit médical suivant l'une des revendications précédentes, **caractérisé en ce qu'**il est prévu au moins 10.000, mais de préférence 16.000 parties (18) de tige par $cm^2$ sur la surface (12, 12') fonctionnelle.

**9.** Produit médical suivant l'une des revendications précédentes, **caractérisé en ce que** les parties (18) de tige sont séparées en forme de spatule à leurs extrémités libres.

**10.** Produit médical suivant l'une des revendications 1 à 10, **caractérisé en ce que** les parties (18) de tige ont un corps (20) de tige ayant la forme d'un hyperboloïde de révolution.

**11.** Produit médical suivant l'une des revendications 1 à 10, 12, **caractérisé en ce que** les parties (18) de tige ont une partie (24) de tête élargie par rapport au corps (20) de la tige, les côtés supérieurs associés à l'adhérence des parties (18) de tête élargies étant sensiblement planes ou légèrement convexes.

**12.** Produit médical suivant l'une des revendications précédentes, **caractérisé en ce que** la partie (18) de tige et/ou le corps (20) de tige a une hauteur de 50 $\mu$m à 150 $\mu$m, de préférence d'environ 90 $\mu$m, et un diamètre de 10 $\mu$m à 40 $\mu$m, de préférence d'environ 30 $\mu$m.

**13.** Produit médical suivant la revendication 13 ou 14, **caractérisé en ce que** les parties (24) de tête élargies par rapport au corps (20) de tige ont un diamètre de 15 $\mu$m à 70 $\mu$m, de préférence d'environ 50 $\mu$m.

10

12

14

II

## Fig.1a

10

15

13    14    16    12

## Fig.1b

12

24
20

24
20

22

22

24
20

18

22

13

X

## Fig.2

Fig.3

Fig.4

Fig.5a

Fig.5b

Fig.6a

Fig.6b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1190723 A2

- US 2005148984 A1